# FASCICULE DE BREVET EUROPEEN

(11) **EP 0 760 859 B1**
(45) Date de publication et mention de la délivrance du brevet: **19.11.2003**
(21) Numéro de dépôt: 95920967.7
(22) Date de dépôt: 19.05.1995
(51) Int. Cl.: C12N 15/74, C12N 15/52, C12N 15/53, C12N 15/60, A61K 39/04

(54) **VECTEURS NAVETTES POUR L'INTRODUCTION D'ADN DANS DES MYCOBACTERIES ET UTILISATION DE CES BACTERIES COMME VACCINS**
VEKTOREN ZUM EINSCHAEUSE VON DNS IN MYKOBAKTERIEN UND VERWENDUNG DIESER BAKTERIEN ALS IMPFSTOFFE
SHUTTLE VECTORS FOR THE INTRODUCTION OF DNA INTO MYCOBACTERIA AND UTILIZATION OF SUCH BACTERIA AS VACCINES

(30) Priorité: 20.05.1994 FR 9406202
(43) Date de publication de la demande: 12.03.1997
(73) Titulaire: INSTITUT NATIONAL DE LA SANTE ET DE LA RECHERCHE MEDICALE, 75654 Paris Cédex 13 (FR); INSTITUT PASTEUR DE LILLE, 59019 Lille Cédex (FR)
(72) Inventeur: ESCUYER, Vincent, 91300 Massy (FR); BAULARD, Alain, 7500 Tournai (BE); BERCHE, Patrick, 92200 Saint-Cloud (FR); LOCHT, Camille, 59830 Wannehain (FR); HADDAD, Nadia, 75012 Paris (FR)
(74) Mandataire: Le Brusque, Maurice
(86) Numéro de dépôt international: FR9500664
(87) Numéro de publication internationale: WO95032296

(56) Documents cités:
- JOURNAL OF BACTERIOLOGY, vol. 172, Novembre 1990 WASHINGTON D.C., US, pages 6557-6567, HERRERO, ET AL. 'Transposon vectors containing non-antibiotic resistance selection markers for cloning and stable chromosomal insertion of foreign genes in Gram-negative bacteria' cité dans la demande
- J BACTERIOL 157 (2).669-672, Février 1984 MEISSNER, P. ET AL. 'PLASMID ENCODED MERCURIC REDUCTASE IN MYCOBACTERIUM -SCROFULACEUM.' cité dans la demande
- PROCEEDINGS OF THE NATIONAL ACADEMY OF SCIENCES OF USA, vol. 85, Septembre 1988 WASHINGTON US, pages 6987-6991, SNAPPER, S. ET AL. 'Lysogeny and transformation in mycobacteria: stable expression of foreign genes' cité dans la demande
- PLASMID, vol. 27, no. 1, Janvier 1992 pages 4-16, MISRA, T.K. 'Bacterial resistances to inorganic mercury salts and organomercurials' cité dans la demande
- PLASMID, vol. 27, no. 1, Janvier 1992 pages 29-40, KAUR, P. & ROSEN, B. 'Plasmid-encoded resistance to arsenic and antimony' cité dans la demande
- MICROBIOLOGY (READING) 141 (4). 1045-1050, Avril 1995 BAULARD, A. ET AL. 'Mercury resistance as a selective marker for recombinant mycobacteria.'

## Description

La présente invention a pour objet des vecteurs navettes pour l'introduction d'ADN dans des mycobactéries.

Elle est en outre relative à l'utilisation comme vaccins de ces mycobactéries dans lesquelles un de ces vecteurs a été introduit.

Le genre Mycobacterium comprend plus de 50 espèces , incluant des pathogènes majeurs pour l'homme, tels que Mycobacterium tuberculosis agent de la tuberculose, Mycobacterium leprae, agent de la lèpre, et Mycobacterium avium, un opportuniste majeur au cours du SIDA. On connaît peu les mécanismes génétiques de la virulence de M. tuberculosis et des autres espèces pathogènes pour l'homme, du fait du faible taux de croissance de ces bactéries et du manque de vecteurs de clonage adéquats, ce qui rend difficile l'introduction d'ADN étranger dans ces microorganismes. Les mycobactéries montrent de plus une résistance particulière à beaucoup d'antibiotiques du fait de la constitution de leur paroi peu perméable à ces molécules. Des récents travaux ont néanmoins montré qu'il était possible d'introduire de l'ADN étranger dans le BCG, dans le but de faire exprimer des antigènes hétérologues à cette bactérie.

Dans le cas de manipulation du BCG, l'utilisation de marqueurs de résistance aux antibiotiques jusqu'ici disponibles chez les mycobactéries, tels que le gène aph3 qui confère un haut niveau de résistance à la kanamycine ou de gènes conférant la résistance à la streptomycine ou à la spectinomycine, n'est pas envisageable , car elle pourrait être à l'origine d'une dissémination de la résistance à ces antibiotiques dans l'environnement, au cas où le BCG recombinant serait utilisé à des fins thérapeutiques ou prophylactiques chez l'homme et chez l'animal. Il est donc de la plus haute importance de pouvoir développer des marqueurs de résistance sans danger pour l'environnement dans le but de sélectionner des mycobactéries recombinantes, qui pourraient être largement utilisées.

Actuellement, un nombre très réduit de marqueurs utilisables dans les mycobactéries est disponible.

Ainsi, Aldovini et al. ( J. Bacterial, 1993, 175, 7282-7289) ont isolé le gène codant pour l'orotidine-5'-monophosphate décarboxylase (OMP-DCase) de Mycobacterium bovis BCG.

Un autre marqueur, le gène cI, codant pour le répresseur du phage L1 a d'autre part été utilisé (demande WO- 90/00594).

Des vecteurs navettes permettant le transfert d'ADN entre Escherichia coli et Mycobacterium, basés sur ces marqueurs ont déjà été décrits. Ainsi, la demande WO-91/13 157 décrit deux vecteurs navettes, pEP2 et pEP3 porteurs respectivement d'un gène de résistance à la kanamycine et à l'hygromycine.

Un de ces vecteurs, le pEP2, est modifié par l'introduction du promoteur de la protéine MBP70 de Mycobacterium, comme le décrit la demande WO-90/10 701.

D'autres vecteurs navettes utilisables dans des mycobactéries et portant des marqueurs de résistance aux antibiotiques , des marqueurs d'auxotrophie ou le gène cI ont été décrits dans la demande WO 90/00594.

Enfin, RANES et al. ( J. Bacteriol. 1990, 172, 2793-2797) ont décrit la construction d'un vecteur navette appelé pRR3 portant un gène de résistance à la kanamycine s'exprimant dans Mycobacterium bovis BCG et Mycobacterium smegmatis.

On connaissait aussi (Meissner et Falkinham, J. Bacteriol, 157, 669-672, 1984) une souche de mycobactérie saprophyte non pathogène résistante au mercure portant un plasmide de 115 MD. Néanmoins ce plasmide, de taille très importante, n'était pas caractérisé. En particulier les gènes impliqués dans la résistance au mercure n'étaient ni localisés ni identifiés. Ce plasmide ne pouvait donc être utilisé comme plasmide navette. De plus, les mycobactéries saprophytes ne font pas partie des mycobactéries pathogènes , ces dernières étant principalement comprises dans les espèces M.tuberculosis, M.bovis et M.leprae.

L'homme du métier désireux de transférer des ADN codant pour des antigènes, obtenus par clonage dans Escherichia coli, dans des mycobactéries en vue de fabriquer des vaccins, se trouvait donc confronté à une absence de vecteurs faciles à manipuler, portant des marqueurs s'exprimant chez E. coli et dans les mycobactéries, et sans danger pour la santé humaine ou animale. En effet, l'utilisation de gènes de résistance à des antibiotiques d'intérêt thérapeutique est à exclure pour les raisons invoquées ci-dessus. L'utilisation de mutants auxotrophes est difficile pour des raisons pratiques de sélection et en outre impose d'obtenir des mutants pour chaque souche de mycobactérie.

Les demandeurs se sont donc attachés à rechercher d'autres marqueurs pouvant être inclus dans des vecteurs navettes sans diminuer de manière excessive la capacité de ces vecteurs à inclure d'autres fragments d'ADN, et conférant aux mycobactéries dans lesquelles ils s'expriment des caractères permettant de les distinguer, d'une manière non ambiguë et facile à mettre en oeuvre, des mycobactéries ne portant pas ces marqueurs.

Les demandeurs ont ainsi mis en évidence de manière surprenante que des vecteurs navettes issus de bactéries Gram-négatives, portant des gènes de résistance à un composé contenant un métal lourd, tel que le mercure inorganique ou des composés mercuriels, s'exprimaient dans des bactéries Gram-positives et pouvaient être utilisés pour le transfert d'ADN entre Escherichia coli et Mycobacterium, et que des bactéries portant ces vecteurs navettes pouvaient être facilement sélectionnées.

La présente invention a donc pour objet un vecteur navette pour l'introduction d'ADN dans des mycobactéries comprenant au moins une origine de réplication fonctionnelle dans lesdites mycobactéries, une autre origine de réplication fonctionnelle dans d'autres bactéries, telles que E.coli, un site de coupure enzymatique permettant l'insertion d'ADN codant pour une protéine susceptible d'être exprimée dans les mycobactéries, caractérisé en ce qu'il porte en plus un gène conférant auxdites mycobactéries la résistance à un composé contenant un métal lourd, tel que le mercure inorganique, un composé mercuriel ou l'arsenic.

On notera que le problème de la recherche de marqueurs pour le transfert de gènes dans des mycobactéries était posé à l'homme du métier depuis un grand nombre d'années, mais ce problème n'avait jamais été résolu de manière satisfaisante.

La solution à ce problème trouvée par les demandeurs, le choix de gènes issus de bactéries Gram-négatives et codant pour une résistance à un métal lourd, est d'autant plus surprenante qu'à priori l'homme du métier aurait pensé que des gènes issus de bactéries Gram-négatives ne s'exprimeraient pas dans des bactéries Gram-positives. En effet, plusieurs études ont montré l'incapacité de gènes issus de bactéries Gram-négatives à s'exprimer dans des Gram-positives (Misra, Plasmid, 27, 4-16, 1992; Robinson et Tuovinen, Microbiological Reviews, Vol.48 n°2, 95-124, 1984)

Les demandeurs ont donc résolu un problème qui se posait de longue date et qui de plus est extrêmement important pour la santé publique. En effet, les mycobactéries, et en particulier M.bovis BCG, sont considérées comme de bons candidats pour la vaccination à l'encontre de diverses maladies, pour autant que l'on arrive à faire exprimer des antigènes étrangers par ces bactéries. La présente invention permet une introduction efficace et sans danger pour la santé humaine de gènes codant pour ces antigènes.

La présente invention s'accompagne donc d'un progrès technique important dans le domaine de la santé humaine.

On remarquera en outre que si l'existence de marqueurs de résistance au mercure était déjà connue, en particulier chez les mycobactéries, l'homme du métier n'avait été en aucune manière incité à intégrer des gènes de résistance au mercure ou à des composés mercuriels dans des vecteurs navettes utilisables chez les mycobactéries. Au contraire, il est notoire que les opérons s'exprimant chez E. coli par exemple, ne s'expriment pas chez les mycobactéries . L'homme du métier n'était donc pas incité à utiliser dans les mycobactéries des gènes s'exprimant habituellement chez E. coli.

En d'autres termes, il n'était en rien évident d'utiliser les gènes de résistance, au mercure ou aux composés mercuriels en particulier, comme gènes de sélection des mycobactéries ayant reçu les vecteurs navettes.

Si l'un des objets de la présente invention est le transfert d'ADN hétérologue dans Mycobacterium, on notera que les vecteurs navettes peuvent aussi être utilisés pour transférer des fragments d'ADN de mycobactéries dans d'autres bactéries telles qu'Escherichia coli afin de faciliter l'étude génétique des mycobactéries. En effet, les mycobactéries, du fait principalement de leur faible vitesse de croissance, sont difficiles à étudier d'un point de vue génétique et le clonage de fragments de leur ADN dans Escherichia coli facilite une telle étude. Les vecteurs navettes objets de la présente invention peuvent ainsi être avantageusement utilisés pour le clonage de l' ADN des mycobactéries et l'étude de ces bactéries.

Ils peuvent aussi être utilisés pour atténuer la virulence de M. tuberculosis et autres mycobactéries virulentes à l'aide de transposons.

La présente invention devrait ainsi accélérer considérablement les progrès de l'analyse génétique des mycobactéries et permettre la manipulation génétique du BCG afin de fabriquer des vaccins multivalents.

On entend dans la présente invention par ADN hétérologue de l'ADN n'appartenant pas à l'espèce de Mycobacterium choisie pour l'exprimer. A titre d'exemple d'ADN hétérologue transférable on citera ceux décrits dans EP 91.401.601.

Les gènes de résistance au mercure et aux composés mercuriels, tels que les composés organomercuriels, sont organisés en opéron.

Six gènes mer ont été décrits jusqu'à présent pour le transport des ions Hg²⁺ dans le cytoplasme et leur dé-toxification.

L'enzyme clef est une mercurique réductase codée par le gène merA réduisant les ions Hg²⁺ en mercure élémentaire Hg^{O}, qui est un composé volatil relativement peu toxique et facilement éliminé par les bactéries.

On connaît aussi le gène merB qui code pour une organo-mercuriel lyase capable de cliver la liaison C-Hg et de transformer le mercure en ion Hg²⁺ qui est ensuite détoxifié par la mercurique réductase décrite ci-dessus.

L'ensemble des gènes de résistance au mercure pouvant être utilisé dans le cadre de la présente invention, ainsi que leurs séquences, peuvent être ceux répertoriés dans la revue de Misra (1992, précédemment cité) ou de Robinson et Tuoniven (1984, précédemment cités) ou par Fellay et al. (1987, Gene, 52, 147-154), Griffin et al. (1987, Proc. Natl. Acad. Sci. USA, 84, 3112-3116) ou Herrero et al.(1990, J. Bacteriol., 172, 6557-6567).

Néanmoins, tout autre gène de résistance au mercure s'exprimant dans les mycobactéries peut être intégré dans les vecteurs navettes objets de la présente invention.

Avantageusement, le vecteur objet de la présente invention comprend soit le gène merA seul, soit les gènes merA et merB, avec les gènes merT et merP.

Dans le premier cas, le vecteur comprend avantageusement l'opéron merA du transposon Tn501. Dans le second cas, le vecteur comprend avantageusement les opérons merA et merB clonés à partir du plasmide pDU1358 isolé à partir de Serratia marcescens (Griffin et al, précédemment cités) et exprimant un large spectre de résistance à l'ion Hg²⁺ et à un composé organomercuriel tel que l'acétate phényl mercurique (PMA).

Avantageusement, un vecteur navette selon l'invention et portant l'opéron merA est le plasmide pMROO1 déposé auprès des Collections de Microorganismes Coordonnées Belges (BCCM) sous le n° LMBP 3046 et un vecteur portant les deux opérons merA et merB est le plasmide pVN2 déposé auprès des BCCM sous le n°LMBP 3047. Ces deux plasmides portent par ailleurs les gènes merT et merP, ainsi que merR pour le pMROO1.

Bien que les gènes de résistance au mercure soient avantageusement utilisés dans la présente invention, on notera néanmoins que tout autre gène de résistance à un métal lourd, tel que l'arsenic, le cadmium ou le plomb peut aussi être utilisé.

Des gènes conférant la résistance à ces autres métaux lourds sont notamment ceux décrits par Silver et Walderhaug (Microbiological Reviews, Vol.56, N°1, 195-228, 1992) Kaur et Rosen (Plasmid 27, 29-40, 1992) et Nies (Plasmid,27, 17-28, 1992).

Afin de conférer la résistance à l'arsenic, on peut utiliser le gène ars. Un vecteur portant un tel gène est le pVN3 déposé auprès des BCCM sous le n°LMPB 3048.

Préférentiellement, le ou les gènes de résistance au composé contenant un métal lourd, ainsi que le site de coupure enzymatique sont portés par un transposon. Une telle construction permet de faciliter l'intégration de l'ADN portant le site de coupure dans lequel a été inséré l'ADN codant pour la protéine que l'on souhaite voir s'exprimer dans la mycobactérie, en particulier dans le cas où la recombinaison homologue est peu efficace.

Comme il a été indiqué ci-dessus, ledit vecteur selon l'invention comprend un site de coupure enzymatique permettant l'insertion d'ADN codant pour une protéine, susceptible d'être exprimé dans les mycobactéries.

La présente invention est donc relative non seulement au vecteur présentant ce site de coupure mais aussi à un vecteur dans lequel a été inséré, au niveau de ce site de coupure, un fragment d'ADN codant pour une protéine hétérologue à l'hôte choisi.

Une telle protéine est avantageusement un antigène susceptible d'induire une réponse immunitaire chez l'homme ou chez l'animal, tel qu'un antigène de M.leprae , un antigène de M.tuberculosis, un antigène du vecteur de la malaria, la toxoïde diphtérique, la toxoïde tétanique, un antigène de Leishmania, un antigène d'une salmonelle, un antigène de M.africanum, de M.intracellulae, de M.avium, un antigène de Trépanoma, de Toxoplasma, Schistosoma, Trypanesoma de Pertussis, un antigène du virus de l'herpès de l'HBV, de l'HCV, de Shigella, de Neisseria, de Borrelia, du virus de la poliomyélite, du virus HIV, ou un antigène d'un venin de serpent, d'insecte ou du Vibrio cholerae ou tout autre antigène, incluant les antigènes provenant de cellules eucaryotes, utile à l'immunoprophylaxie ou à l'immunothérapie.

Les origines de réplication du vecteur navette objet de la présente invention sont toutes origines permettant la réplication d'un vecteur dans E. coli ou dans les mycobactéries. Avantageusement, les origines de réplication dans E.coli et dans les mycobactéries sont celles utilisées dans le plasmide pRR3.

Celles d'autres plasmides tels que pMSC262 et RSF1010 peut aussi être avantageusement utilisées.

On notera néanmoins que si l'origine n'est pas fonctionnelle dans les mycobactéries, ou si elle n'est pas fonctionnelle de manière optimale, le vecteur navette peut s'intégrer dans l'ADN chromosomique des mycobactéries par exemple par transposition ou recombinaison.

Le site de coupure permettant l'intégration dans le vecteur navette du fragment d'ADN codant pour la protéine que l'on souhaite exprimer dans les mycobactéries est tout site de coupure par un enzyme de restriction permettant d'obtenir des bouts collants ou des bouts francs. L'homme du métier choisira un tel enzyme en fonction des conditions de manipulation, telles que la facilité d'utilisation de l'enzyme.

De manière générale, et en particulier pour la fabrication du vecteur objet de la présente invention et pour l'intégration de l'ADN dans ce vecteur, l'homme du métier pourra se référer au manuel technique général suivant: Maniatis et al.1982, Molecular cloning: A. Laboratory Manual; Cold Spring Harbor Laboratory, Cold Spring Harbor N.Y. USA ou à une de ses récentes rééditions.

L'homme du métier pourra se référer en outre à ce manuel pour toute préoccupation intéressant la présente invention.

La présente demande a encore pour objet des mycobactéries pathogènes, en particulier M.bovis, M.leprae, M.tuberculosis,M.avium, M.intracellulare présentant une résistance à un composé contenant un métal lourd tel que le mercure inorganique , un composé mercuriel ou l'arsenic.

De manière générale, la présente invention est relative à des mycobactéries, en particulier pathogènes ou atténuées, portant un vecteur tel que décrit ci-dessus.

Elle est aussi relative aux mycobactéries portant un gène de résistance à un métal lourd et exprimant un gène codant pour un antigène hétérologue.

On entend par antigène hétérologue un antigène ne se trouvant pas habituellement dans la bactérie dans lequel on l'exprime.

Le vecteur peut se trouver dans la bactérie sous une forme non intégrée. Néanmoins, il sera avantageusement au moins en partie intégré dans le chromosome bactérien.

De manière particulièrement avantageuse, les mycobactéries portent un transposon, contenant le gène de résistance au métal lourd et le gène codant pour une protéine susceptible d'être exprimée chez les mycobactéries, inséré dans leur chromosome. Un tel mode de mise en oeuvre est particulièrement avantageux car il diminue les risques de perte d'expression de la protéine qui peuvent avoir lieu plus fréquemment quand les vecteurs ne sont pas intégrés.

D'autres objets de la présente invention sont des compositions pharmaceutiques contenant au moins une mycobactérie telle que définie ci-dessus en association avec un ou plusieurs excipients compatibles et pharmaceutiquement acceptables et des vaccins contenant de telles mycobactéries.

Enfin, un autre objet de la présente invention est une méthode d'introduction d'un gène dans une mycobactérie comprenant au moins une étape d'introduction dans ladite mycobactérie d'un vecteur tel que décrit ci-dessus portant ledit gène et une étape de sélection des mycobactéries dans lesquelles le vecteur a été introduit. De manière avantageuse les mycobactéries dans lesquelles le plasmide a été introduit sont sélectionnées pour leur résistance au mercure ou à un composé mercuriel.

L'introduction du vecteur dans des mycobactéries est avantageusement effectuée par électroporation. Néanmoins, toute autre méthode permettant d'arriver à un tel résultat peut être tout aussi valablement utilisée.

La présente invention est illustrée sans pour autant être limitée par les exemples suivants dans lesquels:
- les figures 1 et 2 représentent respectivement des cartes schématiques des plasmides pMROO1 et pVN2.
- les figures 3 et 4 illustrent la sensibilité de clones de M.smegmatis recombinants et non-recombinants respectivement au PMA et au HgCl₂,
- les figures 5 et 6 illustrent la sensibilité de clones de BCG recombinants et non-recombinants respectivement au PMA et au HgCl₂,
- les figures 7 et 8 illustrent la sensibilité de clones de H37RA recombinants ou non-recombinants respectivement au PMA et au HgCl₂,
- la figure 9 est une carte schématique du plasmide pVN3.

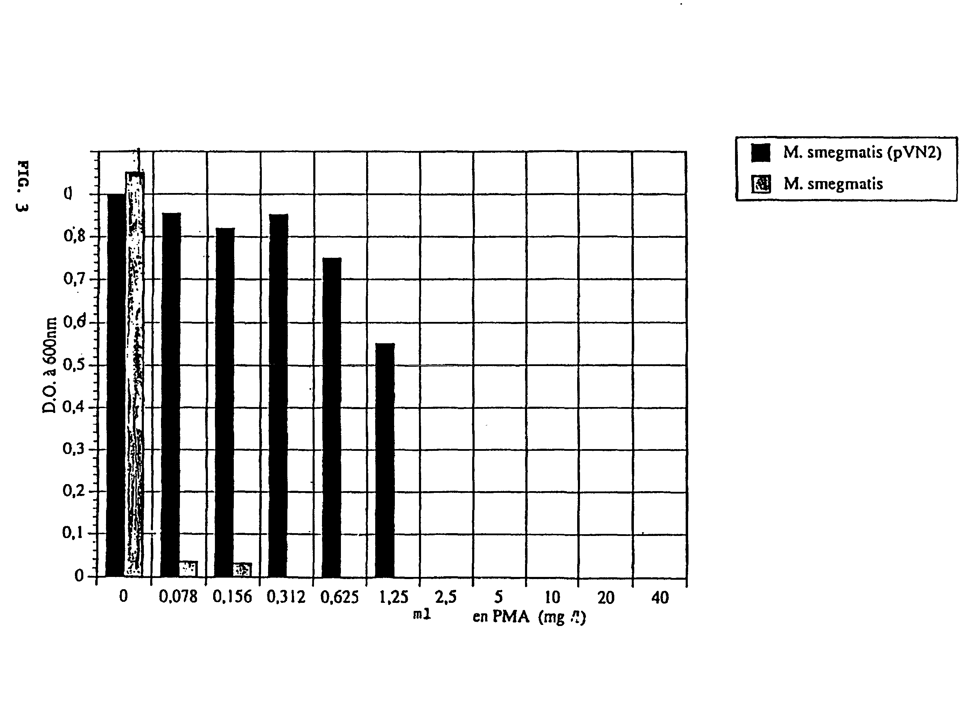

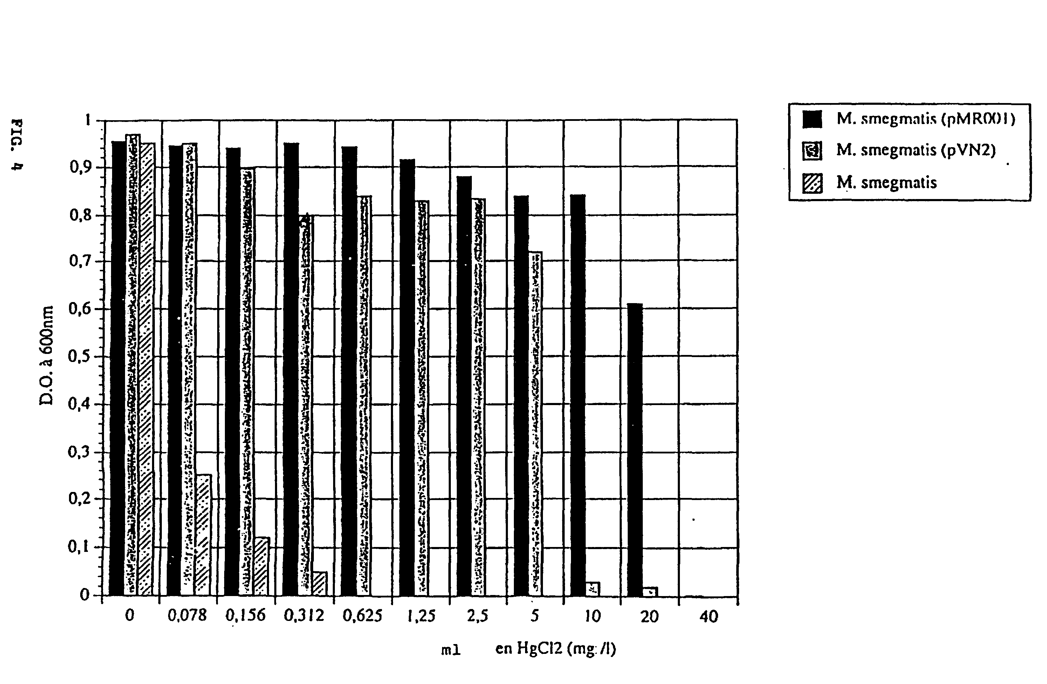

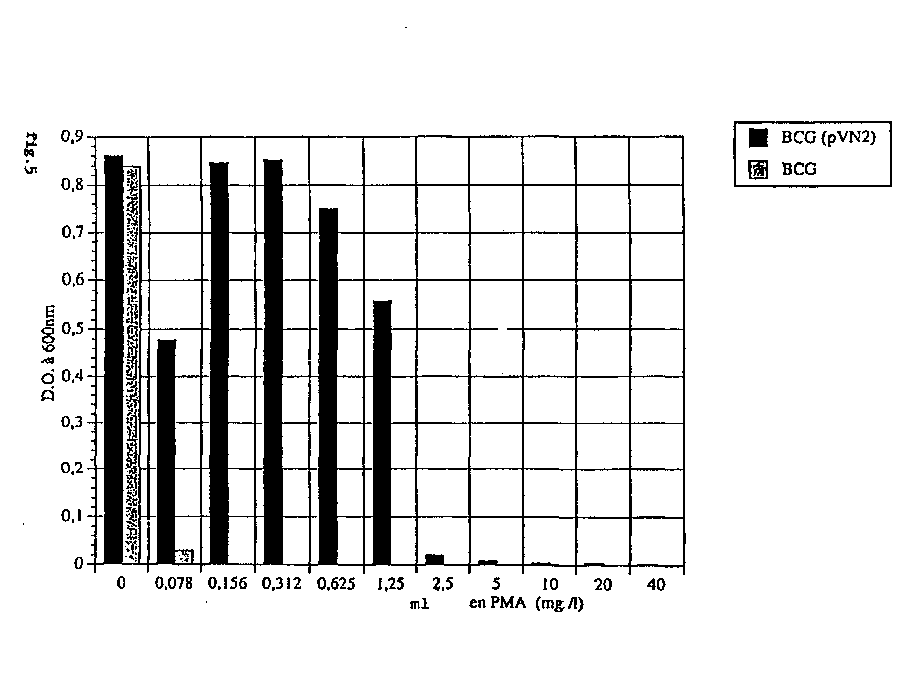

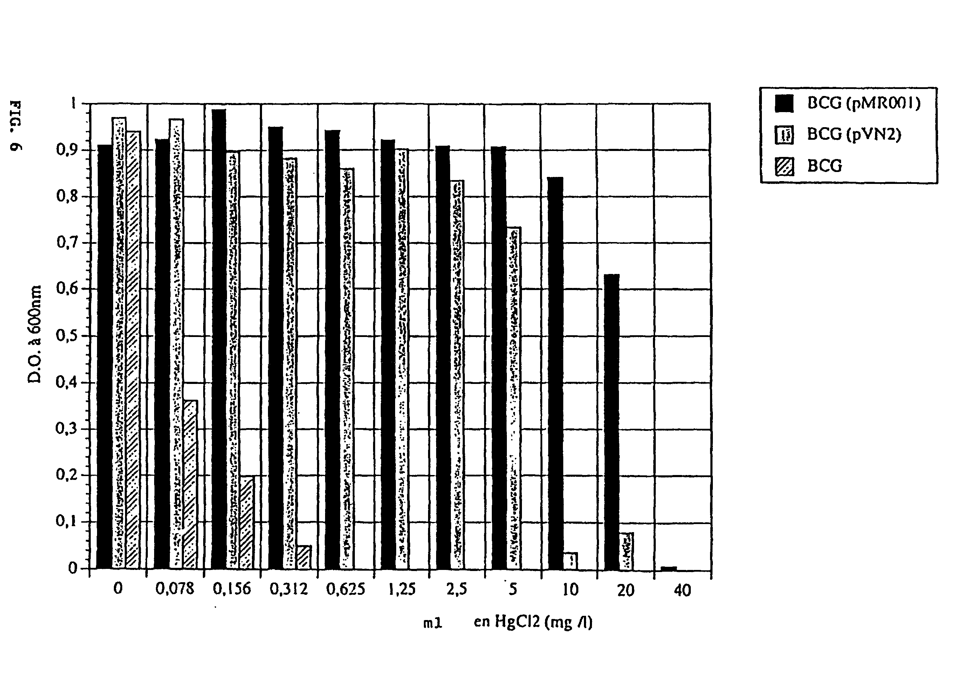

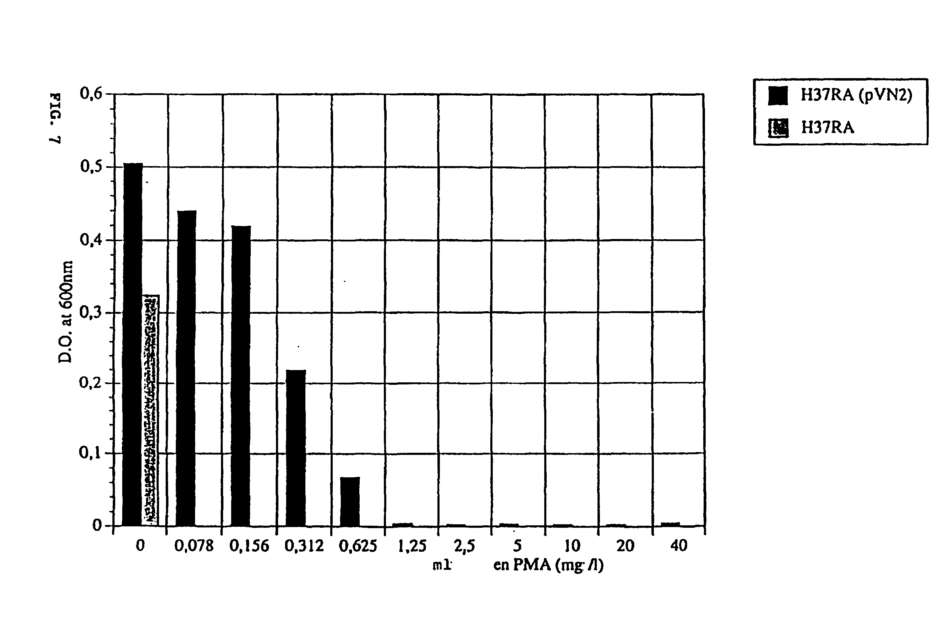

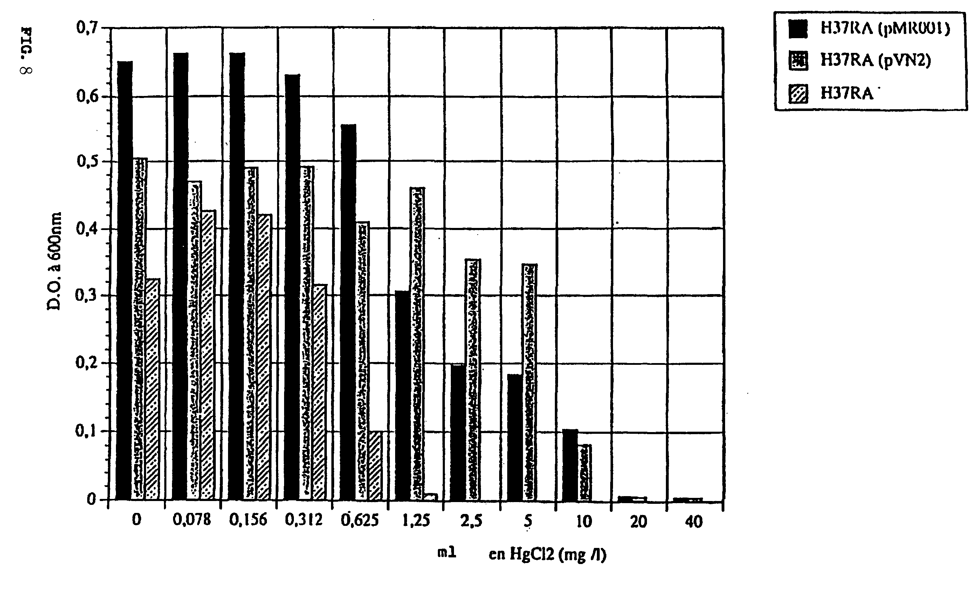

Dans les figures 3 à 8 la croissance des clones est mesurée par la densité optique à 600 nm (en ordonnée) en fonction de la concentration en PMA ou en HgCl₂ (en abscisse).

### EXEMPLES :

### EXEMPLE 1:

### Construction des plasmides navettes portant des gènes de résistance au mercure pMROO1 et pVN2 et leurs transferts dans des mycobactéries.

Deux plasmides portant des opérons mer sont utilisés:
- le pHP45-Ω-Hg (Fellay et al (1987, Gene, 52, 147-154)) contenant l'opéron mer du transposon Tn501 trouvé à l'origine chez Pseudomonas aeruginosa et exprimant seulement la résistance au mercure inorganique, codé par merA,.
- le pLOF-Hg(Herrero et al (1990, J. Bacteriol, 172, 6557-6567)) contenant des gènes de résistance à la fois au mercure et aux composés organomercuriels, incluant merA et merB, clonés à partir du plasmide pDU1358, isolé à l'origine à partir de Serratia marcescens, et exprimant ainsi un large spectre de résistance aux ions Hg²⁺ et aux composés organomercuriels tels que l'acétate phénylmercurique (PMA).

Les opérons mer de ces plasmides ont été introduits séparément dans le vecteur navette pRR3.

Les bactéries, E. coli ou mycobactéries, utilisées dans cet exemple sont mentionnées dans le tableau 1.

### 1°) Fabrication de pMROO1

Le fragment SmaI de 4,3 kb de pH45ΩHg qui contient l'opéron mer de Tn501 est inséré dans le site unique ScaI de pRR3.

Le mélange , après ligation, est utilisé pour transformer E. coli XL1-Blue par électroporation. Les bactéries transformées sont ensuite étalées sur du milieu Luria Broth (LB) complémenté avec 12µg/ml HgCl₂.

Plusieurs colonies exprimant la résistance au mercure apparaissent après une incubation d'une nuit à 37°C, tandis qu'aucune colonie n'est observée quand on étale des bactéries transformées avec le pRR3 seul.

Les plasmides de 10 clones présentant une résistance au mercure sont extraits et analysés. La présence de plasmides recombinants présentant le schéma de restriction attendu est confirmée.

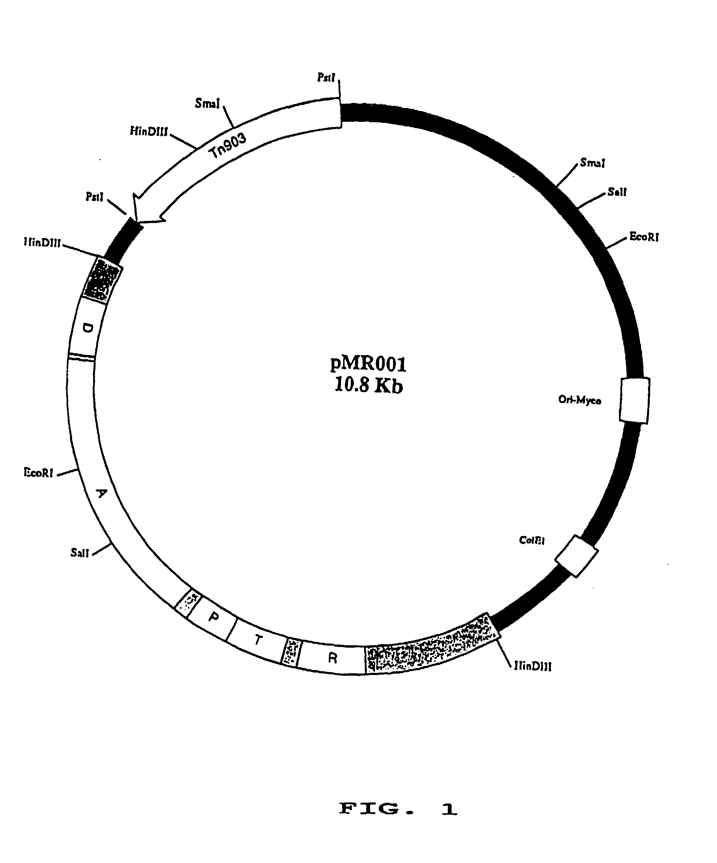

Un plasmide désigné pMR001, et dont la carte schématique de restriction est représentée sur la figure 1, est alors utilisé pour transformer les mycobactéries.

### 2°) Fabrication de pVN2

Une stratégie semblable est mise en oeuvre avec pLOF-Hg, un plasmide portant l'opéron mer de S. marcescens. Le fragment Mlu de 3 kb du pLOF-Hg portant l'opéron mer de S. marcesens a été traité par la DNA polymérase I (Klenow) et inséré ensuite dans le site unique ScaI de pRR3. Les bactéries transformées expriment la résistance au mercure, et on n'observe aucun réarrangement dans les plasmides recombinés.

Un plasmide désigné pVN2 a été ultérieurement utilisé pour transformer les mycobactéries.

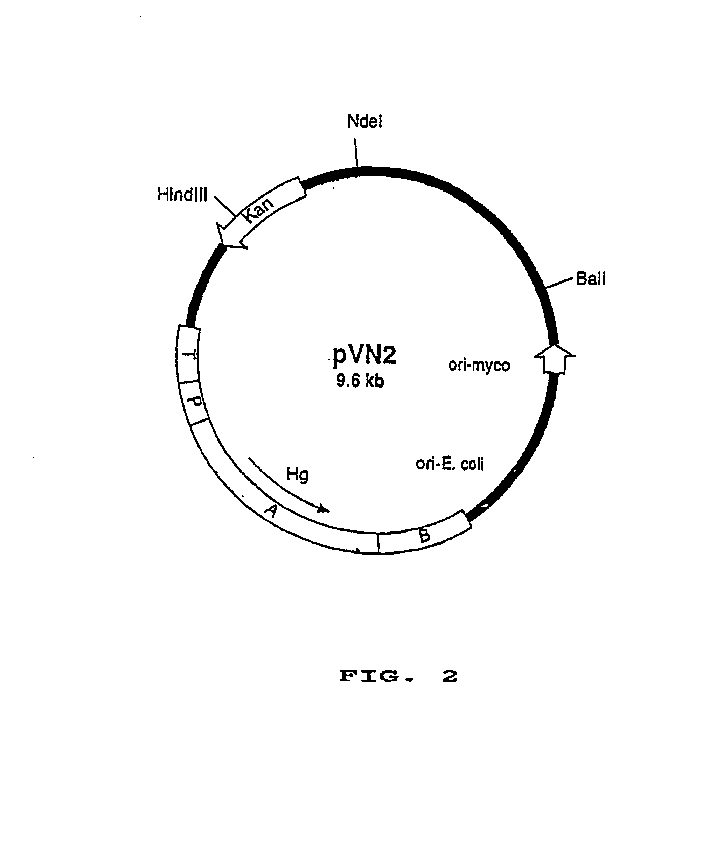

Ce plasmide est représenté de manière schématique sur la figure 2.

### 3°) Electroporation de mycobactéries par pMROO1 et pVN2.

pMROO1 et pVN2, portant la résistance au mercure et la résistance à la kanamycine , ont été utilisés pour électrotransformer M. smegmatis, M.bovis BCG et M.tuberculosis, en utilisant un appareillage d'électroporation (Eurogentec S.A.), tel que décrit par Baulard et al. (1992, Nucleic Acid Research 20, 4105).

Les signaux de transcription-traduction de l'opéron mer de P. aeruginosa pouvant ne pas être reconnus dans les mycobactéries, les bactéries transformées par pMROO1 ont tout d'abord été sélectionnées sur de l'agar Middlebrook 7H10 (Difco, Détroit, MI, USA) complémentés avec de la kanamycine à raison de 20µg/ml.

Après cinq jours, ou deux ou trois semaines, d'incubation à 37°C, selon la mycobactérie receveuse, des colonies recombinantes isolées sont obtenues avec les trois espèces de mycobactéries.

Pour estimer la concentration inhibitrice minimale (CIM), définie comme la plus faible concentration de composés mercuriels à laquelle on n'observe pas de croissance bactérienne, 10 µl d'une suspension de bactéries (10⁸-10⁹ bactéries/ml) sont déposés en gouttes sur du milieu agar Middlebrook 7H10 (Difco) ou 100µl sont inoculés dans des fioles de Roux Middlebrook contenant 10ml de milieu liquide de Sauton complémentés avec des concentrations croissantes de HgCl₂.

Les cultures sont incubées à 37°C durant cinq jours (M. smegmastis) ou deux à trois semaines (M.tuberculosis, M. bovis BCG), jusqu'à ce que la croissance bactérienne soit détectable dans les cultures témoins.

Alors que les mycobactéries témoins montrent une susceptibilité importante à des concentrations de 0,15µg/ml d'HgCl₂ sur milieu solide, les mycobactéries portant le plasmide pMROO1 montrent une augmentation très importante du niveau de résistance à HgCl₂.

Une inhibition complète de la croissance bactérienne n'est observée qu'à des concentrations de 160µg/ml sur milieu solide. Quand la détermination de la concentration inhibitrice minimale (CIM) pour HgCl₂ est effectuée dans du milieu liquide de Sauton, on observe une inhibition complète de la croissance bactérienne à une concentration de 1µg/ml d'HgCl₂ pour les mycobactéries témoins non transformés ( Tableau 2).

Par contre, les mycobactéries portant le plasmide pMROO1 sont toujours capables de croître, à une concentration de 20µg/ml d'HgCl₂.

Les ADN plasmidiques de 12 clones résistant à 15 µg/ml d'HgCl₂ de trois mycobactéries d'espèces différentes, sont ensuite transférés par électroduction à des bactéries E. coli XL1-Blue comme décrit par Baulard (1992, précédemment cités).

Les plasmides des E.coli transformées résistantes au mercure montrent un schéma de digestion normal.

Comme attendu, la résistance au mercure conférée par le plasmide pMR001 est restreinte au mercure inorganique et ne concerne pas les composés organomercuriels.

Pour déterminer si les résistances au mercure du pMROO1 et du pVN2 peuvent être utilisées comme marqueurs de sélection directe pour la transformation des mycobactéries, des mycobactéries M.bovis BCG M. smegmatis et M. tuberculosis électroporées avec l'un des deux plasmides ont été directement étalées sur un milieu agar 7H10 complémenté avec 12µg/ml d'HgCl₂. Après 5 à 15 jours de culture, selon l'espèce, 10³ colonies résistantes par µg de pMROO1 ont été obtenues.

On effectue les mêmes opérations avec le plasmide PVN2 exprimant la résistance au mercure inorganique et aux composés organomercuriels (PMA). La CIM des M.Segmatis, M.Bovis BCG et M.tuberculosis non transformées est inférieure à 0,15µg/ml pour le PMA. Des mycobactéries portant le pVN2 obtenues par électroporation comme décrit ci-dessus poussent sur un milieu agar 7H10 jusqu'à des concentrations de 40µg/ml d'HgCl₂ et 5 µg/ml de PMA. 10³ colonies résistantes par µg apparaissent après 25 jours sur des boîtes d'agar 7H10 contenant 10 µg/ml de PMA.

Dans le milieu liquide de Sauton, l'inhibition de la croissance est observée à une concentration de 0,15 µg/ml de PMA pour les bactéries non transformées des trois espèces de mycobactéries.

Le niveau de résistance au mercure pour les mycobactéries transformées par le PVN2 est estimé à 5 µg/ml de PMA ou 40 µg/ml d'HgCl₂ (tableau 2).

Les résultats obtenus sont résumés sur les figures 3 à 8.

Dans tous les cas, une transformation retour d'Escherichia coli par électroduction confirme la présence du plasmide attendu dans les clones de mycobactéries résistants.

### EXEMPLE 2:

### Construction d'un vecteur navette portant la résistance à l'arsenic.

Le plasmide pLOF/As porte les gènes arsA et arsB provenant du plasmide pR773 qui a été isolé chez diverses entérobactéries. Les deux gènes sont placés sous le contrôle du promoteur de l'aérobactine.

Un fragment de 3,4 kb a été obtenu par digestion de pLof-As par l'enzyme MluI, qui porte arsA, arsB et le promoteur de l'aérobactine. Les extrémités de ce fragment ont été remplies par l'ADN-polymérase I, et il a été ensuite cloné au site unique ScaI du vecteur-navette Escherichia coli-mycobactérie pYUB12. La construction de ce dernier vecteur est décrite dans Snapper et al. (PNAS, 1988, 85: 6987-6991; et Mol. Microbiol., 1990, 4: 1911-1919).

Le plasmide pVN3 confère à Mycobacterium smegmatis une résistance de l'ordre de 10 mg/l au nitrate d'arsenic (AsNO₃).

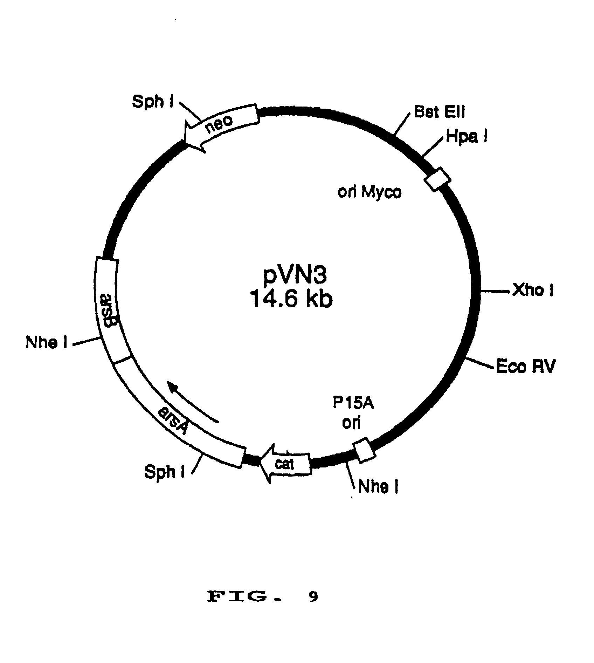

Ce plasmide est représenté schématiquement sur la figure 9 .

### CONCLUSION:

Les résultats obtenus montrent que des vecteurs exprimant la résistance aux métaux lourds, mercure et arsenic, peuvent être utilisés comme marqueurs de sélection pour les mycobactéries recombinantes.

Les vecteurs navettes objets de la présente invention permettent donc un transfert efficace, et facile à mettre en oeuvre et à sélectionner, de gènes hétérologues dans des mycobactéries hôtes capables d'exprimer lesdits gènes hétérologues.

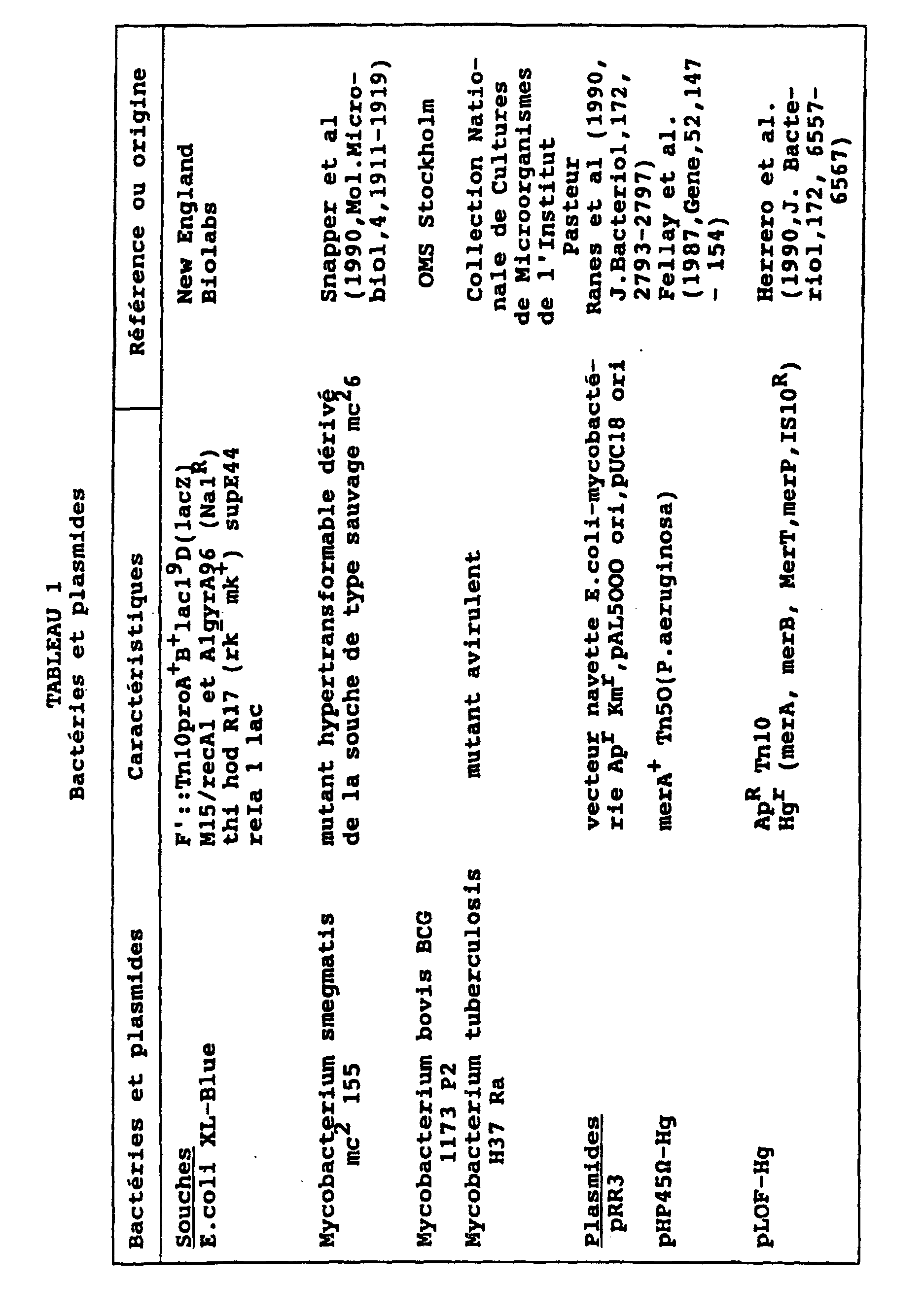

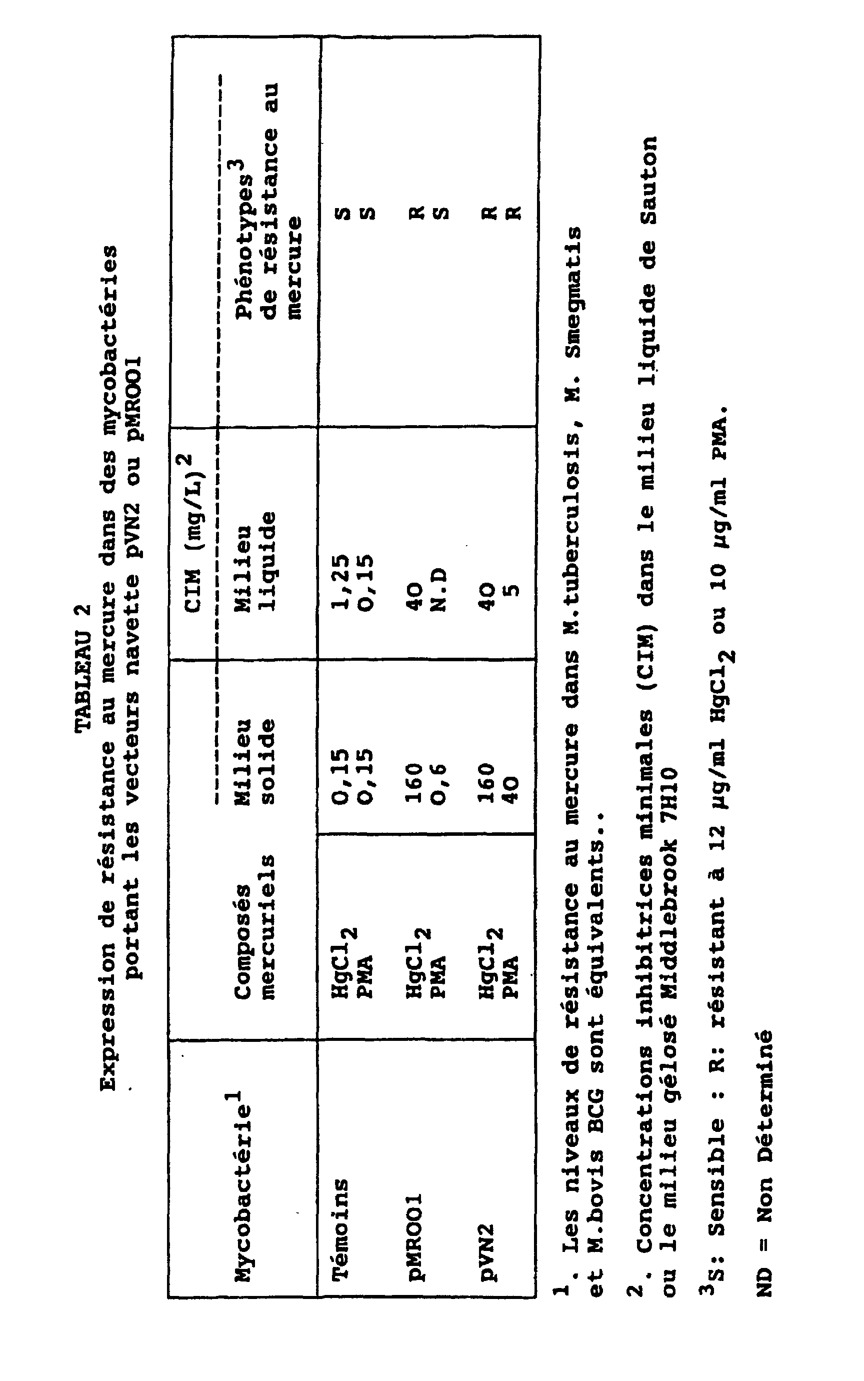

## Revendications

1. Vecteur navette pour l'introduction d'ADN dans des mycobactéries comprenant au moins une origine de réplication fonctionnelle dans lesdites mycobactéries, une autre origine de réplication fonctionnelle dans d'autres bactéries , un site de coupure enzymatique permettant l'insertion d'ADN codant pour une protéine susceptible d'être exprimée dans les mycobactéries, **caractérisé en ce qu'**il porte en plus un gène conférant auxdites mycobactéries la résistance à un composé contenant un métal lourd.

2. Vecteur selon la revendication 1 **caractérisé en ce que** ledit composé est le mercure inorganique ou un composé mercuriel.

3. Vecteur selon l'une des revendications 1 et 2, **caractérisé en ce que** son gène de résistance code pour une mercurique réductase.

4. Vecteur selon l'une des revendications 1 et 2, **caractérisé en ce que** son gène de résistance code pour une organomercuriel lyase.

5. Vecteur selon l'une des revendications 1 à 3, **caractérisé en ce qu'**il comprend le gène merA du transposon Tn501 codant pour une mercurique réductase.

6. Vecteur selon l'une des revendications 1 à 4, **caractérisé en ce qu'**il comprend les gènes merA et merB du plasmide pDU 1358.

7. plasmide pMR001 selon l'une des revendications 1 à 3 et 5 déposé auprès des BCCM sous le n° LMBP 3046.

8. Plasmide pVN2 selon l'une des revendications 1 à 4, et 6 déposé auprès des BCCM sous le n° LMBP 3047.

9. Vecteur selon l'une des revendications 1 à 6 **caractérisé en ce que** le gène de résistance au métal lourd et le site de coupure enzymatique sont portés par un transposon.

10. Vecteur selon la revendication 1 **caractérisé en ce que** ledit métal lourd est l'arsenic.

11. Vecteur selon la revendication 10 **caractérisé en ce que** le gène de résistance à l'arsenic est l'opéron ars.

12. Plasmide pVN3 selon l'une des revendications 10 et 11 déposé auprès des BCCM sous le n° LMBP 3048.

13. Vecteur selon l'une des revendications 1 à 12, **caractérise en ce qu'**il porte un gène s'exprimant dans les mycobactéries et codant pour une protéine susceptible d'indui- re une réponse immunitaire chez l'homme ou chez l'animal.

14. Mycobactérie pathogène ou atténuée présentant une résistance à un composé contenant un métal lourd tel que le mercure inorganique, un composé mercuriel, ou l'arsenic **caractérisée en ce qu'**elle porté un vecteur selon l'une des revendications 1 à 13.

15. Mycobactérie **caractérisée en ce qu'**elle porte au moins la partie contenant le gène de résistance à un composé contenant un métal lourd et le gène codant pour une protéine susceptible d'induire une réponse immunitaire d'un vecteur selon la revendication 13 intégrée dans son chromosome.

16. Mycobactérie **caractérisée en ce qu'**elle porte dans son chromosome un transposon comprenant au moins un gène codant pour la résistance à un composé contenant un métal lourd tel que le mercure inorganique ou un composé mercuriel, et un gène codant pour un antigène.

17. Mycobactérie selon l'une des revendications 15 et 16 **caractérisée en ce qu'**elle est une mycobactérie pathogène.

18. Mycobactérie selon l'une des revendications 14 à 17 **caractérisée en ce qu'** elle est une Mycobacterium bovis BCG, M. smegmatis ou M.tuberculosis.

19. Composition pharmaceutique contenant au moins une mycobactérie selon l'une des revendications 14 à 18, en association avec un ou plusieurs excipients compatibles et pharmaceutiquement acceptables .

20. Vaccin **caractérisé en ce qu'**il contient au moins une bactérie selon l'une des revendications 14 à 18.

21. Méthode d' introduction d'un gène dans une mycobactérie comprenant au moins une étape d'introduction d'un vecteur selon l'une des revendications 1 à 13 portant ledit gène, et une étape de sélection de mycobactéries dans lesquelles le vecteur a été introduit.

22. Méthode selon la revendication 21, **caractérisée en ce que** les mycobactéries dans lesquelles le vecteur a été introduit sont sélectionnées pour leur résistance à un composé contenant un métal lourd, tel que le mercure inorganique ou un composé mercuriel.

## Patentansprüche

1. Shuttlevektor für die Einführung von DNA in Mycobakterien, umfassend wenigstens einen Replikationsstartpunkt, der in den Mycobakterien funktionsfähig ist, einen weiteren Replikationsstartpunkt, der in anderen Bakterien funktionsfähig ist, eine Restriktionsschnittstelle, die den Einsatz von DNA erlaubt, die für ein Protein codiert, das in den Mycobakterien exprimiert werden kann, **dadurch gekennzeichnet, dass** er außerdem ein Gen trägt, das den Mycobakterien Resistenz gegen eine Verbindung, die ein Schwermetall enthält, verleiht.

2. Vektor gemäß Anspruch 1, **dadurch gekennzeichnet, dass** es sich bei der Verbindung um anorganisches Quecksilber oder eine Quecksilberverbindung handelt.

3. Vektor gemäß einem der Ansprüche 1 und 2, **dadurch gekennzeichnet, dass** sein Resistenzgen für eine Quecksilber-Reductase codiert.

4. Vektor gemäß einem der Ansprüche 1 und 2, **dadurch gekennzeichnet, dass** sein Resistenzgen für eine Organoquecksilber-Lyase codiert.

5. Vektor gemäß einem der Ansprüche 1 bis 3, **dadurch gekennzeichnet, dass** er das Gen merA des Transposons Tn501 umfasst, das für eine Quecksilber-Reductase codiert.

6. Vektor gemäß einem der Ansprüche 1 bis 4, **dadurch gekennzeichnet, dass** er die Gene merA und merB des Plasmids pDU 1358 umfasst.

7. Plasmid pMR001 gemäß einem der Ansprüche 1 bis 3 und 5, das bei den BCCM unter der Nr. LMBP 3046 hinterlegt ist.

8. Plasmid pVN2 gemäß einem der Ansprüche 1 bis 4 und 6, das bei den BCCM unter der Nr. LMBP 3047 hinterlegt ist.

9. Vektor gemäß einem der Ansprüche 1 bis 6, **dadurch gekennzeichnet, dass** das Gen für die Resistenz gegen das Schwermetall und die Restriktionsschnittstelle von einem Transposon getragen werden.

10. Vektor gemäß Anspruch 1, **dadurch gekennzeichnet, dass** es sich bei dem Schwermetall um Arsen handelt.

11. Vektor gemäß Anspruch 10, **dadurch gekennzeichnet, dass** es sich bei dem Gen für die Resistenz gegen das Schwermetall um das ars-Operon handelt.

12. Plasmid pVN3 gemäß einem der Ansprüche 10 und 11, das bei den BCCM unter der Nr. LMBP 3048 hinterlegt ist.

13. Vektor gemäß einem der Ansprüche 1 bis 12, **dadurch gekennzeichnet, dass** er ein Gen trägt, das in den Mycobakterien exprimiert wird und für ein Protein codiert, das bei Mensch oder Tier eine Immunantwort induzieren kann.

14. Pathogenes oder abgeschwächtes Mycobakterium, das eine Resistenz gegen eine Verbindung aufweist, die ein Schwermetall, wie anorganisches Quecksilber, eine Quecksilberverbindung oder Arsen, enthält, **dadurch gekennzeichnet, dass** sie einen Vektor gemäß einem der Ansprüche 1 bis 13 trägt.

15. Mycobakterium, **dadurch gekennzeichnet, dass** es in seinem Chromosom integriert wenigstens denjenigen Teil eines Vektors gemäß Anspruch 13 trägt, der das Gen für die Resistenz gegen eine Verbindung, die ein Schwermetall enthält, und das Gen, das eine Immunantwort induzieren kann, enthält.

16. Mycobakterium, **dadurch gekennzeichnet, dass** es in seinem Chromosom ein Transposon trägt, das wenigstens ein Gen, das für die Resistenz gegen eine Verbindung codiert, die ein Schwermetall, wie anorganisches Quecksilber oder eine Quecksilberverbindung, enthält, und ein Gen, das für ein Antigen codiert, umfasst.

17. Mycobakterium gemäß einem der Ansprüche 15 und 16, **dadurch gekennzeichnet, dass** es ein pathogenes Mycobakterium ist.

18. Mycobakterium gemäß einem der Ansprüche 14 bis 17, **dadurch gekennzeichnet, dass** es sich um *Mycobacterium bovis BCG, M. smegmatis* oder *M. tuberculosis* handelt.

19. Pharmazeutische Zusammensetzung, die wenigstens ein Mycobakterium gemäß einem der Ansprüche 14 bis 18 in Verbindung mit einem oder mehreren verträglichen und pharmazeutisch annehmbaren Trägersubstanzen enthält.

20. Impfstoff, **dadurch gekennzeichnet, dass** er wenigstens ein Bakterium gemäß einem der Ansprüche 14 bis 18 enthält.

21. Verfahren zur Einführung eines Gens in ein Mycobakterium, umfassend wenigstens einen Schritt der Einführung eines Vektors gemäß einem der Ansprüche 1 bis 13, der das genannte Gen trägt, und einen Schritt der Selektion von Mycobakterien, in die der Vektor eingeführt wurde.

22. Verfahren gemäß Anspruch 21, **dadurch gekennzeichnet, dass** die Mycobakterien, in die der Vektor eingeführt wurde, anhand ihrer Resistenz gegen eine Verbindung, die ein Schwermetall, wie anorganisches Quecksilber oder eine Quecksilberverbindung, enthält, selektiert werden.

## Claims

1. Shuttle vector for inserting DNA in mycobacteria comprising at least one origin of functional replication in said mycobacteria, another origin of functional replication in other bacteria, an enzyme cutting site allowing the insertion of DNA coding for a protein capable of being expressed in the mycobacteria, **characterized in that** it also carries a gene providing on said mycobacteria resistance to a compound containing a heavy metal.

2. Vector according to claim 1, **characterized in that** said compound is inorganic mercury or a mercurial compound.

3. Vector according to one of claims 1 and 2, **characterized in that** its resistance gene codes for a mercuric reductase.

4. Vector according to one of claims 1 and 2, **characterized in that** its resistance gene codes for an organomercurial lyase.

5. Vector according to one of claims 1 to 3, **characterized in that** it comprises the gene merA of the transposon Tn501 coding for a mercuric reductase.

6. Vector according to one of claims 1 to 4, **characterized in that** it comprises the gene merA and MerB of the plasmid pDU 1358.

7. Plasmid pMR001 according to one of claims 1 to 3 and 5, filed at the BCCM under No. LMBP 3046.

8. Plasmid pVN2 according to one of claims 1 to 4 and 6, filed at the BCCM under No. LMBP 3047.

9. Vector according to one of claims 1 to 6, **characterized in that** the gene of resistance to heavy metal and the enzyme cutting site are carried by a transposon.

10. Vector according to claim 1, **characterized in that** said heavy metal is arsenic.

11. Vector according to claim 10, **characterized in that** the gene of resistance to arsenic is the operon ars.

12. Plasmid pVN3 according to one of claims 10 and 11, filed at the BCCM under No. LMBP 3048.

13. Vector according to one of claims 1 to 12, **characterized in that** it carries a gene expressing itself in the mycobacteria and coding for a protein capable of inducing an immune response in the human and in the animal.

14. Pathogenic or attenuated mycobacterium having a resistance to a compound containing a heavy metal such as inorganic mercury, a mercurial compound or arsenic, **characterized in that** it carries a vector according to one of claims 1 to 13.

15. Mycobacterium **characterized in that** it carries at least the part containing the gene of resistance to a compound comprising a heavy metal and the gene coding for a protein capable of inducing an immune response of a vector, according to claim 13 integrated in its chromosome.

16. Mycobacterium **characterized in that** it carries in its chromosome a transposon comprising at least one gene coding for resistance to a compound containing a heavy metal such as inorganic mercury or a mercurial compound, and a gene coding for an antigen.

17. Mycobacterium according to one of claims 15 and 16, **characterized in that** it is a pathogenic mycobacterium.

18. Mycobacterium according to one of claims 14 to 17, **characterized in that** it is a Mycobacterium bovis BCG, M. smegmatis or M. tuberculosis.

19. Pharmaceutical composition containing at least one mycobacterium according to one of claims 14 to 18, in association with one or more compatible and pharmaceutically acceptable excipients.

20. Vaccine **characterized in that** it contains at least one bacterium according to one of claims 14 to 18.

21. Method of introducing a gene in a mycobacterium comprising at least one introduction step of a vector according to one of claims 1 to 13 carrying said gene, and a step of selecting the mycobacteria in which the vector was introduced.

22. Method according to claim 21, **characterized in that** the mycobacteria in which the vector has been introduced are selected for their resistance to a compound containing a heavy metal such as inorganic mercury or a mercurial compound.
